# EUROPEAN PATENT SPECIFICATION

(11) **EP 1 226 251 B1**
(45) Date of publication and mention of the grant of the patent: **21.01.2009**
(21) Application number: 00989738.0
(22) Date of filing: 01.11.2000
(51) Int. Cl.: C12N 15/12, C07K 14/705, C07K 16/28, C12Q 1/68, G01N 33/50

(54) **CpG RECEPTOR (CpG-R) AND METHODS RELATING THERETO**
CpG REZEPTOR (CpG-R) UND DARAUF BEZOGENE METHODEN
RECEPTEUR DE CpG (CpG-R) ET PROCEDES CORRESPONDANTS

(30) Priority: 02.11.1999 US 163157 P; 24.11.1999 US 167389 P
(43) Date of publication of application: 31.07.2002
(73) Proprietor: Novartis Vaccines and Diagnostics, Inc., Emeryville, CA 94608 (US)
(72) Inventor: MACKICHAN, Mary, Lee, San Francisco, CA 94110 (US)
(74) Representative: Hallybone, Huw George
(86) International application number: PCT/US2000/041735
(87) International publication number: WO 2001/032877

(56) References cited:
- WO-A-00/31540
- WO-A-98/50547
- WO-A-98/52581
- DATABASE EMBL SEQUENCE DATABSE [Online] Hinxton, UK; 30 June 2000 (2000-06-30) T.H. CHUANG AND R.J. ULEVITCH: "Cloning and characterization of a sub-family of human toll-like receptors: hTLR7, hTLR8 and hTLR9" XP002168477 & EUR. CYTOKINE NETW. , vol. 11, no. 3, 2000, pages 372-378,
- KRIEG A M ET AL: "THE ROLE OF CPG DINUCLEOTIDES IN DNA VACCINES" TRENDS IN MICROBIOLOGY,ELSEVIER SCIENCE LTD., KIDLINGTON,GB, vol. 6, no. 1, January 1998 (1998-01), pages 23-26, XP000857633 ISSN: 0966-842X
- SPARWASSER ET AL: "Bacterial DNA and immunostimulatory CpG oligonucleotides trigger maturation and activation of murine dendritic cells" EUROPEAN JOURNAL OF IMMUNOLOGY,DE,WEINHEIM, vol. 28, no. 6, June 1998 (1998-06), pages 2045-2054, XP002131393 ISSN: 0014-2980
- HEMMI HIROAKI ET AL: "A Toll-like receptor recognizes bacterial DNA." NATURE (LONDON), vol. 408, no. 6813, 2000, pages 740-745, XP002168474 ISSN: 0028-0836
- T. KAISHO AND S. AKIRA ET AL.: "Dentritic-cell function in Toll-like receptor- and MyD88-knockout mice" TRENDS IN IMMUNOLOGY, vol. 22, no. 2, February 2001 (2001-02), pages 78-83, XP002168475 ELSEVIER SCIENCE LTD., AMSTERDAM, NL
- L. O'NEILL: "Specificity in the innate response: pathogen recognition by Toll-like receptor combinations" TRENDS IN IMMUNOLOGY, vol. 22, no. 2, February 2001 (2001-02), page 70 XP002168476 ELSEVIER SCIENCE LTD., AMSTERDAM, NL

## Description

### CROSS REFERENCE TO RELATED APPLICATIONS

This application claims priority benefit under 35 U.S.C. §119(e) to Application Serial No. 60/163,157 filed November 2, 1999 and to Application Serial No. 60/167,389 filed November 24, 1999,

### FIELD OF THE INVENTION

The present invention is related generally to immune responses and to the identification of a protein and pathway for signaling an immune response, and specifically to identification of a CpG receptor (CpG-R).

### BACKGROUND OF THE INVENTION

The innate immune system of mammals recognizes and responds to molecular features characteristic of pathogenic organisms. Various portions of the pathogen, such as surface proteins, particular cell wall components and certain nucleotide sequences, may be recognized and trigger a variety of immune responses. It has long been known that cells carry a variety of receptors and membrane bound proteins that recognize these foreign elements and trigger the cascade known as the immune response. Two broad classifications or types of responses are well known: humoral, or antibody-mediated immunity; and cell-mediated immunity. Certain pathogens or conditions may be effectively controlled by primarily an antibody-mediated reaction, while other conditions or pathogens require a vigorous cellular response to mediate a host defense.

Adjuvants are compounds that are capable of potentiating the innate immune response. Adjuvants can potentiate both humoral and cellular immunity. For some conditions or diseases such as, for example, those caused by the human immunodeficiency virus or hepatitis C virus, it is particularly desirable to increase the innate cell-mediated immune response by the administration of an adjuvant.

Unmethylated CG dinucleotide sequences, which are commonly found in bacterial DNA but not in mammalian DNA, have been found to stimulate the innate immune system (reviewed in Lipford et al., Trends Microbiol.,1998, 6, 496-500, Carson et al., J. Exp. Med., 1997, 186, 1621-2*,* and Krieg et al., Trends Microbiol.,1998, 6, 23-7). Exposure to unmethylated CG oligonucleotides or motifs in natural DNA or in synthetic oligonucleotides directly activates antigen presenting cells (APCs), dendritic cells and macrophages (Jakob et al., J. Immunol.,1998, 161, 3042-9*,* Sparwasser et al., Eur. J. Immunol.,1998, 28, 2045-54*,* Sparwasser et al., Eur. J. Immunol.,1997, 27, 1671-9, Stacey et al., J. Immunol., 1996, 157, 2116-22, and Jakob et al., Int. Archives Allergy Immunol., 1999, 118, 457-461), and B cells (Krieg et al., Nature, 1995, 374, 546-9). NK and T cells are also activated by exposure to oligonucleotides containing CpG motifs, although some of these effects may be mediated indirectly by cytokines made by other cell types (Bendigs et al., Eur. J. Immunol., 1999, 29, 1209-18, Sun et al., J. Exp. Med., 1998,188, 2335-42*,* and Ballas et al., J. Immunol., 1996, 157, 1840-5).

Inclusion of oligonucleotides containing CpG motifs as adjuvants in experimental vaccines, by either direct incorporation into plasmid DNA or by co-administration of synthetic CpG oligonucleotides with protein antigens, yields increased antibody titers, especially of the IgG2a subtype. Oligonucleotides containing CpG motifs have also been shown to be potent stimulators of cytolytic T cell activity, which is characteristic of the cell-mediated immune response (reviewed in Brazolot-Millan et al., Proc. Natl. Acad. Sci. USA, 1998, 95, 15553-8, Davis et al., J. Immunol., 1998, 160, 870-6, Davis Mt. Sinai J. Med., 1999, 66, 84-90, and McCluskie et al., J. Immunol.,1998, 161, 4463-6). The ability of oligonucleotides containing CpG motifs to induce expression of Th1 cytokines, especially interleukin-12 (IL-12) and interferon-γ (IFN-γ), likely accounts for the strong Th1 bias of the immune response observed when oligonucleotides containing CpG motifs are used to adjuvant diverse antigens, notably hepatitis B. Th1 type cell-mediated immunity is believed necessary for protection against many vaccine targets currently under study, such as the aforementioned human immunodeficiency virus. Oligonucleotides containing CpG motifs may also have therapeutic applications in treating microbial infections, as CpG oligonucleotide treatment has been shown to confer protection against pathogens such as Listeria and Leishmania even when administered without accompanying antigen (Zimmermann et al., J. Immunol., 1998, 160, 3627-30, Lipford et al., Eur. J. Immunol.,1997, 27, 3420-6, and Walker et al., Proc. Natl. Acad. Sci. USA, 1999, 96, 6970-6975). In addition, CpG oligonucleotide treatment protects immature B cells from apoptosis. Further CpG oligonucleotide activity is somewhat sequence and methylation specific in that unmethylated CG with 5' flanking purines and 3' flanking pyrimidines have the greatest effect. Pretreatment with CpG oligonucleotides has a protective effect in allergic and LPS-induced asthma models (Schwartz, J. Inmunol., 163, 224 and Sur et al., J. Immunol., 162, 6284) and can reduce IgE production *in vitro* by human peripheral blood mononuclear cells from atopic patients.

The mechanism by which CpG oligonucleotides interact with cellular receptors and initiate intracellular signaling are unknown. DNA containing CpG motifs, in the context of oligomers or plasmid DNA, is taken up via an endocytic pathway and accumulates in endosomes *(*Hacker et al., EMBO J.,1998, 17, 6230-40, Yi et al., J. Immunol., 1998, 161, 4493-7*,* Yi et al., J. Immunol., 1998, 160, 4755-61*,* and Macfarlane et al., J. Immunol.,1998,160, 1122-31). This uptake does not appear to be sequence specific, as it can be competed with by DNA lacking CpG motifs *(Hacker et al., supra*)*.* There is some evidence that responses to oligonucleotides containing CpG motifs may require cellular uptake (Krieg *et al.,* **1995**, *supra*) and endosomal acidification, as chloroquine and other blockers of endosome maturation can inhibit cellular and molecular responses to oligonucleotides containing CpG motifs. The exact mechanism of this inhibition is unresolved, as is the subcellular location of any CpG oligonucleotide-related protein, or putative CpG receptor.

Although the mechanism by which cells recognize oligonucleotides containing CpG motifs has not been previously described, it is known that CpG oligonucleotides activate multiple intracellular signaling pathways in macrophages and B cells. Specifically, the stress-activated MAP kinases, JNK and p38, and their transcription factor targets, AP-1 and ATF2, are phosphorylated in response to CpG oligonucleotide treatment (Hacker et al., EMBO J.,1998, 17, 6230-40, and Yi et al., J. Immunol.,1998,161, 4493-7)*.* The pathway leading to nuclear translocation of NF-κB, which involves a kinase cascade leading to phosphorylation and degradation of inhibitory subunits, the IκBs (see Zandi et al., Mol. Cell. Biol., 1999, 19, 4547-51)*,* is also activated by oligonucleotides containing CpG motifs (Yi et al., J. Immunol.,1998,160, 1240-5*,* and Yi et al., J. Immunol., 1998, 160, 4755-61)*.*

Other immunostimulatory agents are known to have similar effects. Monophosphoryl lipid A (MPL) is known to those skilled in the art to induce a Th1 lymphocyte response (Ullrich et al., in Monophosphoryl Lipid A as an Adjuvant in Vaccine Design: The Subunit and Adjuvant Approach, Powell and Newman, Eds., 1995, pp.495-523). In macrophages, the MAPK and NF-κB pathways are activated by other agents, notably the bacterial endotoxin, lipopolysaccharide (LPS). Nuclear NF-κB, often in combination with other transcription factors like AP-1, is required for induction of transcription of many immune response genes, including costimulatory molecules and cytokines (reviewed in Baldwin, Annu. Rev. Immunol., 1996, 14, 649-83).

In mice, genetic evidence links immune responsiveness to bacterial endotoxin to a member of the Toll/IL-1 receptor family, Toll-like receptor (TLR) 4. Experiments with human TLRs implicate TLR2 and/or TLR4 in LPS responses (reviewed in Qureshi et al., Trends in Genetics, 1999, 15, 291-294). More recently, additional bacterial products, including peptidoglycan and mycobacterial lipoproteins, have also been shown to act through members of the Toll receptor family (Yoshimura et al., J. Immunol., 1999, 163, 1-5, Brightbill et al., Science, 1999, 285, 732-736, Aliprantis et al., Science, 1999, 285, 736-739, and Hirschfeld et al., J. Immunol., 1999, 163, 2382-2386). Toll-related receptors have been conserved across millions of years of evolution; in insects and even plants they are genetically associated with responses to a variety of pathogens, including bacteria, fungi, and at least one virus (reviewed in Hoffmann et al., Science, 1999, 284, 1313-1318). It is unknown whether oligonucleotides containing CpG motifs similarly act through Toll-related receptors, or whether a putative CpG receptor might contain a Toll Homology Domain (THD).

WO 98/50547 discloses nucleic acids encoding nine human receptors, designated DNAX toll-like receptors 2-10 (DTLR 2-10), homologous to the Drosophila toll receptor and the human IL-1 receptor.

Because of the potential usefulness of these CpG oligonucleotides as stimulators of various immune responses, there is considerable interest in the isolation, characterization, and mechanisms of actions of CpG-R and signaling pathway. It is an aim of the invention, therefore, to characterize and identify CpG-R, to differentiate it from other proteins or receptors that may lead to similar immune responses, and develop useful methods that will allow the design of novel CpG-R ligands that can retain the immunostimulatory properties of oligonucleotides containing CpG motifs, while having more desirable pharmacological properties.

### SUMMARY OF THE INVENTION.

The present invention is described in the claims.

### BRIEF DESCRIPTION OF THE DRAWINGS

Figure 1A is a bar graph representing activation of NF-κB in RAW 264.7 macrophages, where RAW 264.7 cells were transfected with a κB-driven luciferase reporter gene and treated 24 hours later with interleukin-1 (IL-1; 20 ng/ml), MPL (1 µg/ml), LPS (1 µg/ml) or a phosphorothioate CpG oligonucleotide (4 µM) for 6 hours.
Figure 1B is a graph representing CpG oligonucleotide-induced κB-luciferase (κB-Luc) activation, where RAW 264.7 cells were transiently transfected with a κB-luciferase reporter plasmid and were treated 24 hours later with the indicated concentration of phosphorothioate oligonucleotide containing CpG motif (0.25-8 µM) or a control oligonucleotide (4 µM) for 6 hours.
Figure 1C is a graph representing kinetics of κB-luciferase activation by CpG oligonucleotides or LPS, where RAW 264.7 cells were transiently transfected with κB-Luc and treated 24 hours later with a CpG oligonucleotide (4 µM; open circles), or LPS (1 µg/ml; filled circles).
Figure 2A graph showing that a dominant-negative mutant MyD88 (MyD88*lpr*) inhibits CpG oligonucleotide-induced activation of κB-luciferase, where RAW 264.7 cells were transfected with κB-Luc (1 µg) alone (black bars) or with a plasmid encoding MyD88*lpr* at two different ratios, 10:1 (gray bars) or 1:1 (white bars).
Figure 2B is a bar graph representing activation of NF-κB and inhibition by MyD88*lpr* is dependent on unmethylated CG sequences, where RAW 264.7 macrophages were transfected with kB-Luc alone (black bars) or κB-Luc plus MyD88*lpr* (white bars) (1:1 plasmid ratio).
Figure 3A is a bar graph showing a representative luciferase assay of NIH-3T3 cells transfected with plasmids encoding MyD88 dominant-negative constructs.
Figure 3B a bar graph showing a representative luciferase assay of RAW 264.7 cells transfected with plasmids encoding MyD88 dominant-negative constructs.
Figure 4 is a bar graph representing Toll-like receptor 4 (TLR4) independence for APC responses to CpG oligonucleotide treatment, where immature bone marrow derived dendritic cells (BMDDC) from wild-type (Balb/c; black bars) and TLR4 mutant mice (C3H/HeJ; white bars) were grown in GM-CSF, as described above, for 6 days and then treated overnight with CpG oligonucleotide (5 µM), a control oligonucleotide (5 µM), or LPS (1 µg/ml), or left unstimulated.

### DESCRIPTION OF PREFERRED EMBODIMENTS

The present invention is based upon the surprising discovery of the identification of a specific CpG-receptor (CpG-R). It is further based on the surprising discovery that the CpG-R, or complex containing the same, contains a THD. Discovery of the presence of a THD within the receptor has permitted testing for the necessity of known Toll receptors for the CpG responses and the further characterization of the CpG-R. The present invention is directed to, *inter alia,* a CpG-R that can be a single polypeptide, or a complex of a plurality of polypeptides, and can optionally contain additional components such as, for example, polysaccharides, lipids, and the like. The CpG-R, or complex containing the same, preferably comprises a THD and interacts with the MyD88 adapter protein. Further, the CpG-R may bind to CpG oligonucleotides.

The practice of the present invention will employ, unless otherwise indicated, conventional methods of virology, immunology, microbiology, molecular biology and recombinant DNA techniques within the skill of the art. Such techniques are explained fully in the literature. See, e.g., Sambrook, et al., Molecular Cloning: A Laboratory Manual (2nd Edition, 1989); DNA Cloning: A Practical Approach, Vols. I & II (D. Glover, ed.); Methods In Enzymology (S. Colowick and N. Kaplan eds., Academic Press, Inc.); Fundamental Virology, 2nd Edition, Vols. I & II (B.N. Fields and D.M. Knipe, eds.), Remington's Pharmaceutical Sciences, 18th Edition (Easton, Pennsylvania: Mack Publishing Company, 1990); Handbook of Experimental Immunology, Vols. I-IV (D.M. Weir and C.C. Blackwell, eds., 1986, Blackwell Scientific Publications); Handbook of Surface and Colloidal Chemistry (Birdi, K.S., ed, CRC Press, 1997) and Seymour/Carrahers Polymer Chemistry (4th edition, Marcel Dekker Inc., 1996).

As used herein, the term "CpG-R" refers to a CpG receptor, which can be a single polypeptide or a complex of a plurality of polypeptides, and can optionally contain additional components such as, for example, polysaccharides, lipids, and the like. The CpG-R can be a protein involved in the CpG signaling cascade or can be a receptor for binding CpG oligonucleotides.

As used herein, the term "activity" refers to any activity, or cascade of activities, that is associated with CpG oligonucleotide binding or signaling, such as those described above.

As used herein, the term "antibody" is meant to, without limitation, refer to complete, intact antibodies, Fab fragments and F(ab)₂ fragments thereof, and chimeric antibodies.

As used herein, the phrase "oligonucleotide comprising at least one CpG motif" or "CpG oligonucleotide" refers to a polynucleotide, preferably an oligonucleotide, comprising at least one unmethylated CG dinucleotide sequence. Oligonucleotides comprising at least one CpG motif can comprise multiple CpG motifs.

As used herein, the phrase "CpG motif" refers to an unmethylated dinucleotide portion of an oligonucleotide which comprises a cytosine nucleotide followed by a guanosine nucleotide. 5-methylcytosine can also be used in place of cytosine.

As used herein, the term "homologous" refers to nucleotide or amino acid sequences characterised by a sequence identity of at least about 70%, more preferably at least about 80%, more preferably at least about 90%, and most preferably at least about 95% to the entire nucleotide or amino acid sequence encoding CpG-R, or to at least a portion of CpG-R. Homologous amino acid sequences include those amino acid sequences that encode conservative amino acid substitutions. Sequence identity can be determined by, for example, the Gap program (Wisconsin Sequence Analysis Package, Version 8 for Unix, Genetics Computer Group, University Research Park, Madison WI), using the default settings, which uses the algorithm of Smith and Waterman (Adv. Appl. Math., 1981,2,482-489.

As used herein, the term "about" means ± 10% of the value it modifies.

As used herein, the term "modulates" means an increase or decrease in the amount or effect of a particular activity or protein.

Nucleic acid molecules comprising novel nucleotide sequences-encoding CpG-R are described herein. The nucleic acid molecules are preferably either RNA or DNA, but can contain both RNA and DNA monomers or peptide nucleic acid monomers. The nucleic acid molecule can be single stranded or double stranded. The monomers of the nucleic acid molecules can be linked via conventional phosphodiester bonds or modified bonds, such as, for example, phosphorothioate bonds, and the like. In addition, the sugar moieties of the monomers can be modified by, for example, addition of 2' substitutions which help confer nuclease resistance and/or cellular uptake. The nucleic acid molecule can also comprise a nucleotide sequence complementary to at least a portion of the nucleotide sequence that encodes CpG-R. Preferably, the nucleic acid molecule comprises a nucleotide sequence complementary to the entire sequence, but can comprise a nucleotide sequence complementary to a portion of the entire sequence. The nucleic acid molecule can also comprise a nucleotide sequence homologous to the nucleotide sequence that encodes CpG-R, and can be at least about 70% homologous, determined as above-mentioned, more preferably at least about 80% homologous, more preferably at least about 90% homologous, and most preferably at least about 95% homologous to the entire sequence encoding CpG-R or to any portion thereof.

A wide variety of alternative cloning and *in vitro* amplification methodologies are well known to those skilled in the art. Examples of these techniques are found in, for example. Berger et al., Guide to Molecular Cloning Techniques, Methods in Enzymology 152 Academic Press. Inc., San Diego, CA (Berger).

Vectors are used herein either to amplify DNA or RNA encoding CpG-R in order to express DNA which encodes CpG-R. Preferred vectors include, but are not limited to, plasmids, phages, cosmids, episomes, viral particles or viruses, and integratable DNA fragments. Preferred viral particles include, but are not limited to, adenoviruses, parvoviruses, herpesviruses, poxviruses, adeno-associated viruses, Semliki Forest viruses, vaccinia viruses, and retroviruses. Preferred expression vectors include, but are not limited to, pcDNA3 (Invitrogen) and pSVL (Pharmacia Biotech), pGEM vectors (Promega), pPROEXvectors (LTI, Bethesda, MD), Bluescript vectors (Stratagene), pQE vectors (Qiagen), pSE420 (Invitrogen), and pYES2 (Invitrogen).

Preferred expression vectors are replicable DNA constructs in which a DNA sequence encoding CpG-R is operably connected to appropriate control sequences capable of effecting the expression of the CpG-R in an appropriate host cell or organism. DNA regions are operably connected when they are functionally positioned with respect to each other. Control sequences include, but are not limited to, a promoter, an operator, a ribosomal binding sequence, and transcription/translation termination sequences.

Preferred vectors preferably contain a promoter which is recognised by the host organism. The promoter sequences of the present invention may be either prokaryotic, eukaryotic or viral. Examples of suitable prokaryotic sequences include the P_{R} and P_{L} promoters of bacteriophage lambda (The bacteriophage Lambda, Hershey, A. D., Ed., Cold Spring Harbor Press, Cold Spring Harbor, NY (1973), and Lambda II, Hendrix, R. W., Ed., Cold Spring Harbor Press, Cold Spring Harbor, NY (1980), ); the trp, recA, heat shock, and lacZ promoters of *E*. *coli* and the SV40 early promoter (Benoist, et al. Nature, 1981, 290, 304-310,. Additional promoters include, but are not limited to, mouse mammary tumor virus, long terminal repeat of human immunodeficiency virus, maloney virus, cytomegalovirus immediate early promoter, Epstein Barr virus, rous sarcoma virus, human actin, human myosin, human hemoglobin human muscle creatine, and human metalothionein. Moreover, appropriate expression vectors can include a marker that allows the screening of the transformed host cells. Expression vectors can be prepared by standard methodology.

Suitable host cells for expression of the polypeptides of the invention include, but are not limited to, prokaryotes, yeast, and eukaryotes. Suitable prokaryotic cells include, but are not limited to, bacteria of the genera *Escherichia, Bacillus, Salmonella, Pseudomonas, Streptomyces,* and *Staphylococcus.* Suitable eukaryotic cells include, but are not limited to, insect cells, HeLa cells, Chinese hamster ovary cells (CHO cells), African green monkey kidney cells (COS cells), and murine 3T3 fibroblasts. Suitable yeast cells include, but are not limited to, the genera *Saccharomyces, Pichia,* and *Kluveromyces.* The polypeptides of the invention can also be expressed using a baculovirus expression system (Luckow et al., Bio/Technology, 1988, 6, 47, Baculovirus Expression Vectors: A Laboratory Manual, O'Rielly et al. (Eds.), W.H. Freeman and Company, New York. 1992, and U.S. Patent No. 4,879,236, In addition, the MAXBACJ complete baculovirus expression system (Invitrogen) can, for example, be used for production in insect cells. Propagation of such cells in cell culture is a routine procedure as described in, for example, Tissue Culture, Academic Press, Kruse and Patterson, eds. (1973).

One aspect of the present invention is directed to the use of an isolated polypeptide encoded by a nucleic acid molecule described above as a CpG receptor. In preferred embodiments of the invention, the isolated polypeptide comprises an amino acid sequence encoding CpG-R. Alternatively, the polypeptide is a fragment of the polypeptide encoding CpG-R. Alternatively, the polypeptide comprises an amino acid sequence homologous to CpG-R or a fragment thereof. A polypeptide having an amino acid sequence which has at least about 70% sequence identity or homology, determined as above-mentioned, at least about 80% sequence identity or homology, preferably about 90% sequence identity or homology, more preferably about 95% sequence identity or homology and most preferably about 98% sequence identity or homology to CpG-R, is contemplated as being included in the present invention. A preferred homologous polypeptide comprises at least one conservative amino acid substitution compared to native CpG-R. Other preferred homologous polypeptides comprises two, three, four, five, six, seven, eight, nine, or up to ten conservative amino acid substitutions compared to native CpG-R. The polypeptides can be expressed in host cells as fusion proteins which may include regions from heterologous proteins. The polypeptides of the invention also can include regions from the same protein but which differ from the naturally-occurring polypeptide in sequence. In addition, homologous CpG-R polypeptide comprises those polypeptides having at least about 70% functional homology, at least about 80% functional homology, preferably about 90% functional homology, more preferably about 95% functional homology and most preferably about 98% functional homology compared to CpG-R. Thus, it is to be understood that the present invention includes proteins homologous to, and having essentially at least one biological property (functional homology) that is substantially similar to a biological property of CpG-R.

The polypeptides of the invention encoding CpG-R can be isolated, for example, by screening recombinant expression libraries or sequence databases, or the like, for the ability to bind CpG oligonucleotides, for comprising a THD domain, and for the ability to interact with MyD88 adapter protein. The MyD88 adapter protein is required for signaling from receptors for products, such as lipopolysaccharides and lipoproteins, as well as for interieukin-1. The polypeptides of the present invention are preferably provided in an isolated form, are preferably substantially purified, and most preferably are purified to homogeneity. Host cells are preferably lysed and the polypeptide is recovered from the lysate of the host cells. Alternatively, the polypeptide is recovered by purifying the cell culture medium from the host cells, preferably without lysing the host cell. The polypeptides can be recovered and purified from recombinant cell cultures by well-known methods, including ammonium sulfate or ethanol precipitation, anion or cation exchange chromatography, hydroxylapatite chromatography and lectin chromatography.

The polypeptides of the invention can be used to generate antibodies against the same and used to screen for compounds that modulate the activity of CpG-R or CpG oligonucleotides. Preferably, the antibody binds to an epitope within CpG-R. The antibodies can be monoclonal or polyclonal. Hybridomas that produce antibodies that bind to the polypeptides of the invention, and the antibodies themselves, are useful in the isolation and purification of the polypeptides. In addition, antibodies may be specific inhibitors of CpG-R activity. Antibodies that specifically bind to the polypeptides of the invention can be used to purify the protein from natural sources or through recombinant technology using well known techniques and readily available starting materials. Methods of making antibodies are known to persons skilled in the art. For techniques for preparing monoclonal antibodies, see e.g. Stiites et al (eds.), Basic and Clinical Immunology (4th ed), Lange Medical Publications, Los Altos, CA, . Production of antibodies, Fab fragments and F(ab)₂ fragments are described in, for example, Harlow, E. and D. Lane (1988) ANTIBODIES: A Laboratory Manual, Cold Spring Harbor Laboratory, Cold Spring Harbor, NY.

Another aspect of the present invention is directed to methods of identifying compounds that bind to either nucleic acid molecules or polypeptides encoding CpG-R comprising contacting CpG-R, or a nucleic acid molecule encoding the same, with a compound, and determining whether the compound binds CpG-R, or a nucleic acid molecule encoding the same. Binding can be determined by binding assays which are well known to those skilled in the art, including but not limited to, gel-shift assays, Western blots, radiolabeled competition assay, co-fractionation by chromatography, co-precipitation, ELISA, and the like, which are described in, for example, Current Protocols in Molecular Biology, 1999, John Wiley & Sons, NY, . The CpG-R polypeptide or nucleic acid molecule used in such a test can either be free in solution, attached to a solid support, attached to a cell surface or located within the cell.

Another aspect of the present invention is directed to methods of identifying compounds that modulate signaling activity of CpG-R comprising contacting CpG-R with a compound, and determining whether the compound modifies activity of CpG-R The activity in the presence of the test compound is measured and compared to the activity in the absence of the test compound. Where the activity of the sample containing the test compound is higher than the activity in the sample lacking the test compound, the compound will have increased activity. Where the activity of the sample containing the test compound is lower than the activity in the sample lacking the test compound, the compound will have inhibited activity. The CpG-R used in such a test can either be free in solution in the presence of suitable substrates, attached to a cell surface, or located within a cell.

Compounds that bind to and/or modulate CpG-R have utility in, for example, vaccine adjuvants promoting cell-mediated immune responses, antibacterials (e.g. protection from Listeria infection), tumor immunotherapy, allergy treatment (e.g. suppressing IgE in human PBMC, shifting from Th2 to Th1), and as anti-inflammatory agents (e.g. for use in cystic fibrosis, sepsis, heart disease, chlamydia, inflammatory bowel disease, arthritis, and multiple sclerosis).

The invention is further illustrated by way of the following examples which are intended to elucidate the invention. The foregoing examples are meant to illustrate the invention and are not to be construed to limit the invention in any way.

### EXAMPLES

### Example 1: General Methodology

### Animals and cell lines

Bone marrow was harvested from female mice 6-12 weeks old of various genotypes (C57B1/6, Balb/c; Charles River, or C3H/HeJ, Jackson Labs). Bone marrow cells were used fresh or were frozen in fetal calf serum (FCS: Summit) containing 10% dimethylsulfoxide and stored at -80°C. Bone-marrow macrophages (BMMO) were prepared as described in Current Protocols in Immunology, *supra.* Briefly, fresh or frozen bone marrow cells were cultured in RPMI, 10% heat inactivated (30 minutes, 56°C) fetal calf serum (FCS), 2 mM L-glutamine, 100 µg/ml streptomycin. 100 units/ml penicillin, 50 µM β-mercaptoethanol and 100 U/ml recombinant macrophage colony stimulating factor (M-CSF; R&D Systems). After 24 hours, nonadherent cells were removed to a new dish, and culture continued for 7 days to produce a macrophage monolayer. Nonadherent BMDDCs were produced by culture in the same medium supplemented with GM-CSF (200 U/ml; Preprotech) rather than M-CSF. The RAW 264.7 mouse macrophage cell line (originally derived from a Balb/c mouse) was obtained from the American Type Culture Collection (ATCC). RAW 264.7 cells are cultured in DMEM with 10% heat inactivated FCS, L-glutamine, streptomycin, penicillin, and 1 µM sodium pyruvate.

### Reagents

Cell cultures were treated as indicated with the following reagents: K235 *E*. *Coli* LPS, gel filtration purified (Sigma), monophosphoryl lipid A from *S*. *minnesota* R595 (MPL; RIBI Immunochem Research, Inc.), sonicated 10 minutes prior to addition. Oligonucleotides with phosphorothioate backbones were synthesized by Oligos Etc (Wilsonville, OR); CpG oligonucleotide sequence, tccatgacgttcctgacgtt (SEQ ID NO: 1); control non-CpG oligonucleotide tccaggacttctctcaggtt (SEQ ID NO:2). Recombinant mouse interleukin-1β (IL-1) (Endogen) and interleukin-18 (IL-18) (Biosource) were also used to stimulate RAW264.7 cells in some experiments. Additional CpG oligonucleotides that can be used in the present invention include, but are not limited to, oligonucleotides that comprise nucleotide sequences such as, for example, tccatgacgttcctgacgtt (SEQ ID NO:3), ataatcgacgttcaagcaag (SEQ ID NO:4), ggggtcaacgttgagggggg (SEQ ID NO:5), tctcccagcgtgcgccat (SEQ ID NO:6), gagaacgctcgaccttcgat (SEQ ID NO:7), tccatgtcgttcctgatgct (SEQ ID NO: 8), tccatgacgttcctgatgct (SEQ ID NO: 9), gctagacgttagcgt (SEQ ID NO:10), atcgactctcgagcgttctc (SEQ ID NO:11), gaaccttccatgctgttccg (SEQ ID NO:12), gctagatgttagcgt (SEQ ID NO: 13), tcaacgtt (SEQ ID NO: 14), gcaacgtt (SEQ ID NO:15), tcgacgtc (SEQ ID NO: 16), tcagcgct (SEQ ID NO:17), tcaacgct (SEQ ID NO:18), tcatcgat (SEQ ID NO:19), tcttcgaa (SEQ ID NO:20), tgactgtgaacgttcgagatga (SEQ ID NO:21), tgactgtgaacgttagcgatga (SEQ ID NO:22), tgactgtgaacgttagagcgga (SEQ ID NO:23), gtttgcgcaacgttgttgccat (SEQ ID NO:24), atggcaacaacgttgcgcaaac (SEQ ID NO:25), cattggaaaacgttcttcgggg (SEQ ID NO:26), ccccgaagaacgttttccaatg (SEQ ID NO:27), attgacgtcaat (SEQ ID NO:28), ctttccattgacgtcaatgggt (SEQ ID NO:29), and tccatacgttcctgacgtt (SEQ ID NO:30).

### Immunoblotting

Following stimulation, BMMO or RAW 264.7 cells were lysed in 1% Triton X-100, 50 mM Tris, 62.5 mM EDTA (pH 8.0) with CompleteJ protease inhibitor cocktail (Boehringer Mannheim) (Triton lysis buffer). Lysates were boiled in reducing sample buffer, separated on 10% polyacrylamide gels using the NuPAGEJ Bis-Tris electrophoresis system (Novex). Nitrocellulose membranes were probed with antibodies against IκB-α, phosphorylated- IκB-α, (New England BioLabs), or anti-IL-18 (Santa Cruz Biotechnology) according to manufacturer's instructions, and visualized with enhanced chemiluminescence (Amersham). *κB-Luc assay*

RAW 264.7 cells were seeded into 6-well plates (Coming) at a density of 3 x 10⁵ cells per well 24 hours prior to transfection. Plasmids used in transfection were pNF-κB-Luc (Clontech) and pCR3.V64-Met-Flag-MyD88*lpr* (kindly provided by Jurgen Tschopp; described in (Burns et al., J. Biol. Chem.,1998, 273,12203-9). Total plasmid DNA concentration was normalized across all wells by addition of empty vector (pCMVKm2, Chiron). Cells were transfected with stated concentrations of DNA in Opti-MEM I (Gibco BRL) with 10 ml of LipofectAMINE (Gibco BRL) per well according to manufacturer's instructions. Cells were incubated with the transfection mixture 3 hours at 37°C, then culture media was replaced and cells were allowed to recover overnight. The following day transfected cells were treated in culture media at 37°C, 5%CO₂ with phosphorothioate oligonucleotides, MPL, LPS, or cytokines at indicated concentrations and times. Cells were washed once with cold phosphate buffered saline (PBS), and lysed with Reporter Lysis BufferJ (Promega). Luciferase activity in lysate supernatants was determined using Microlite 2 plates (Dynex) and a ML 3000 Luminometer (Dynatech) all according to manufacture's instructions. For detection of FLAG-*MyD88lpr* expression, the insoluble pellet from transfected RAW 264.7 cells was resuspended in Triton lysis buffer and sonicated 10 minutes prior to addition of sample buffer and boiling. After separation and transfer to nitrocellulose, membranes were immunoblotted using FLAG M2 (Sigma) antibody.

### Flow cytometry

BMDDC were treated overnight with adjuvants as indicated, washed, resuspended in cold PBS/2% FBS containing FcBlock (0.25 µg/10⁶ cells; Pharmingen), and FITC- and PE-conjugated antibodies (1 µg/10⁶ cells) were added 5 minutes later. Cells were incubate with antibodies on ice 30 minutes, washed, and analyzed by flow cytometry on a FACScan (Becton-Dickenson). Only cells from BMDDC cultures staining positive for CD11c and having forward- and side-scatter properties of live cells were included in the analysis. Changes in CD86 expression are assessed based on geometric mean fluorescence of CD86-FITC staining of the live CD11c cells treated with adjuvants normalize to the geometric mean CD86-FITC fluorescence of untreated BMDDC cultures.

### Example 2: CpG Oligonucleotides Activate MAPK Pathways And NF-κB

Several biochemical responses of mouse BMMO and dendritic cells and the mouse macrophage cell line RAW 264.7 to oligonucleotides containing or lacking CpG motifs were compared. As a model for the effects of CpG motifs within oligonucleotides, an oligonucleotide referred to as 1826 (Chu et al., J. Exp. Med.,1997,186, 1623-31), whose activity in mice has been extensively reported in the literature, was used (Bachmaier et al., Science,1999, 283, 1335-9). The CpG and control oligonucleotides were each 20-mers synthesized with a phosphorothioate backbone, which enhances DNA stability *(*Agrawal et al., Proc. Natl. Acad. Sci. USA, 1991, 88, 7595-9).

Macrophages were differentiated from bone marrow cells of C57B1/6 mice as described in Example 1 and then transferred to low serum medium lacking M-CSF for 4 hours. Lysates were prepared from cells treated 30 minutes with medium alone, a phosphorothioate-modified CpG oligonucleotide (1 µM), a control oligonucleotide lacking CG sequences (1 µM), or MPL (100 ng/ml), and immunoblotting was performed with an antibody recognizing phosphorylated ERK1 and ERK2. RAW 264.7 macrophages were similarly treated for 15 minutes along with an additional treatment with LPS (100 ng/ml), and immunoblotted for phosphorylated ERK1 and ERK2. Immunoblotting of whole-cell extracts from the bone-marrow derived APCs or RAW 264.7 cells demonstrated activation of multiple mitogen-activated protein kinase (MAPK) pathways, including ERK1/2, JNK and p38, by CpG oligonucleotides. Phosphorylation of ERK, JNK and/or the JNK substrate *c-jun,* and of the p38 MAPK substrate ATF2, was observed in all three cell types within an hour of stimulation with the CpG oligonucleotide but not the control lacking CpG sequences (data not shown). These results indicate rapid activation of JNK and p38 pathways by CpG oligonucleotides. The positive result for ERK1/2 MAPK activation in RAW 264.7 cells and BMMO are in contrast to observations in another mouse macrophage cell line, J774, where ERK phosphorylation was not detected in CpG oligonucleotide-treated cells.

The status of the NF-κB pathway in BMMO and RAW 264.7 cells treated with a panel of adjuvants, including the CpG and control oligonucleotides was also examined. BMMO were treated for 1.5 hours with LPS (1 µg/ml), MPL (1 µg/ml), a phosphorothioate-modified CpG oligonucleotide (4 µM) or a control phosphorothioate oligonucleotide (non-CpG; 4 µM). Whole cell lysates were prepared and immunoblotted for IκBα, as described above. Degradation of the NF-kB inhibitory protein IκBα within 1.5 hours in BMMO treated with LPS, MPL or the CpG oligonucleotide was observed, but not with the control oligonucleotide (data not shown).

Lysates were also prepared from untreated RAW 264.7 cells or cells treated with a phosphorothioate CpG oligonucleotide (4 µM) for 5, 10, 20 and 60 minutes as well as a control oligonucleotide lacking CpG motifs for 60 minutes. The lysates were then resolved by electrophoresis, and immunoblotted with antisera to phosphorylated IκBα and total IκBα. IκBα phosphorylation and degradation in RAW 264.7 cells showed similar specific activation by a CpG oligonucleotide. This activation was detected at the level of IκBα phosphorylation within 10 minutes of CpG oligonucleotide addition, and degradation of IκBα was notable at 20 minutes. Although IκBα was resynthesized in CpG oligonucleotide-treated RAW 264.7 cells within one hour, much of the IκBα was phosphorylated, suggesting continued targeting for rapid degradation (data not shown).

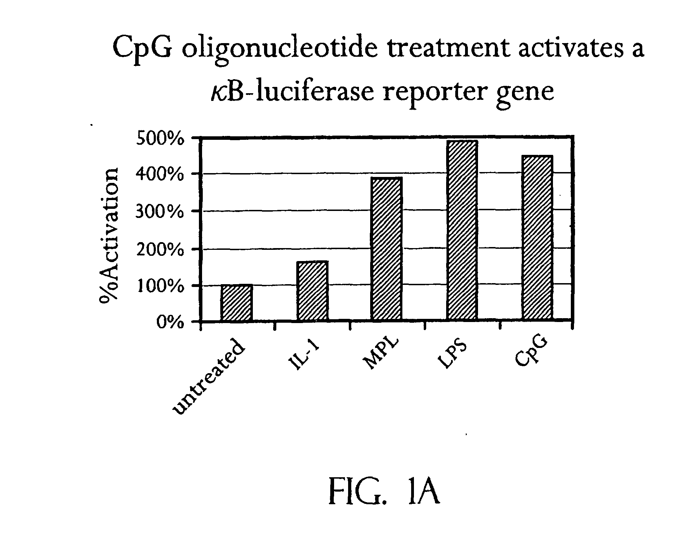

To directly evaluate κB-dependent transcription induced by CpG oligonucleotide or MPL treatment, RAW 264.7 macrophage were transfected with an NF-κB-inducible reporter plasmid encoding luciferase under the control of multiple copies of the kappa light chain enhancer (κB-Luc) and the following day treated with adjuvants or cytokines. In particular, RAW 264.7 cells were transfected with a kB-driven luciferase reporter gene and treated 24 hours later with IL-1 (20 ng/ml), MPL (1 µg/ml), LPS (1 µg/ml) or a phosphorothioate CpG oligonucleotide (4 µM) for 6 hours. Luciferase activity in cell lysates was quantified by luminometry, and results were normalized to the luciferase signal in untreated transfected cells. As shown in Figure 1A, addition of the CpG oligonucleotide to RAW 264.7 cells induced κB-dependent luciferase expression, to a level similar to that induced by MPL or LPS. Activation of the κB reporter gene by the CpG oligonucleotide was slightly weaker than that seen in response to MPL or LPS, both potent activators of NF-κB in macrophages. IL-1 was a poor activator of κB-Luc in RAW 264.7 cells (Figure 1A), as was IL-18 (data not shown).

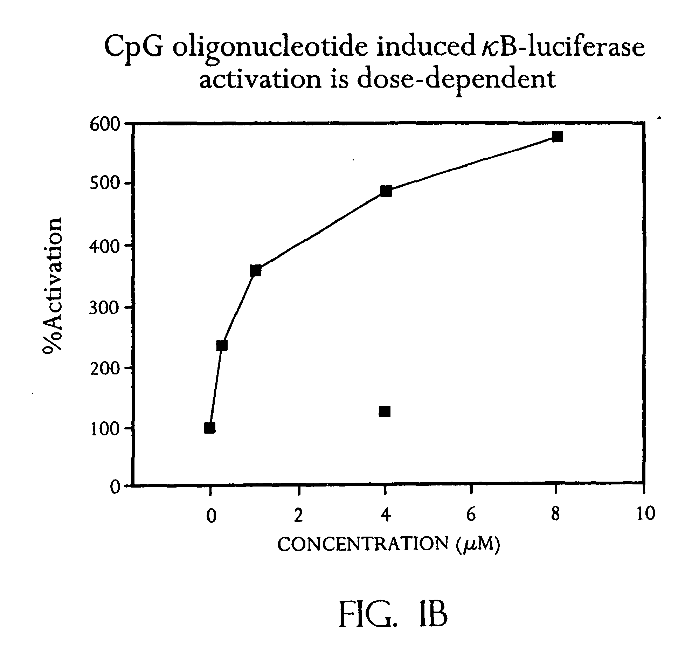

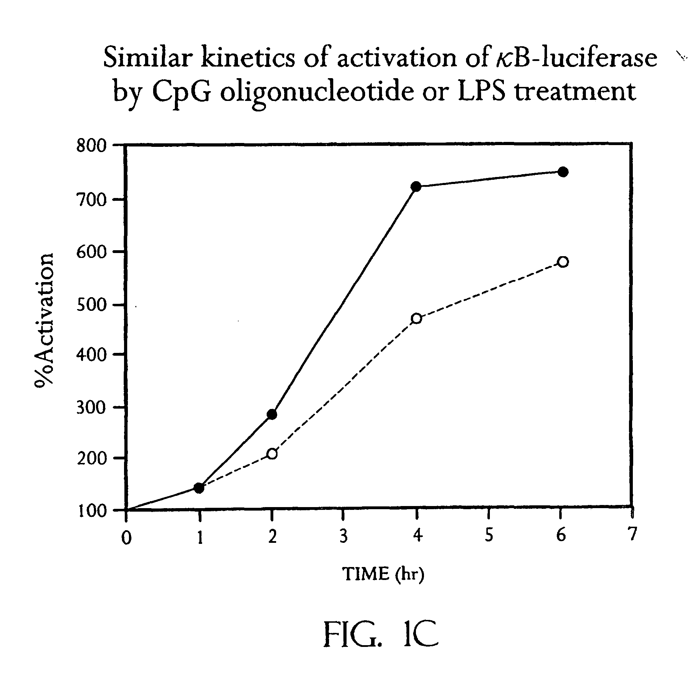

The effects of CpG oligonucleotide on κB-dependent luciferase expression were dose- and time-dependent (see Figures 1B and 1C). In regard to Figure 1B, RAW 264.7 cells transiently transfected with a κB-luciferase reporter plasmid were treated 24 hours later with concentrations of phosphorothioate CpG oligonucleotide concentrations ranging from 0.25-8 µM, or a control oligonucleotide (4 µM) for 6 hours. Activation was detected at CpG oligonucleotide concentrations as low as 0.25 µM (Figure 1B), but not at the lowest concentration tested (0.05 µM) (data not shown). Data shown in Figure 1B are average luciferase activity in lysates from duplicate wells normalized to luciferase levels in lysates from untreated transfected wells on the same 6-well tissue culture plate. Activation of the reporter gene by CpG oligonucleotide treatment was maximal at 8 µM (Figure 1B) and was not further enhanced at a 10 µM dose (data not shown). In regard to Figure 1C, RAW 264.7 cells transiently transfected with a kB-Luc and treated 24 hours later with a CpG-containing oligonucleotide (4 µM; open circles), or LPS (1 µg/ml; filled circles). Results shown in Figure 1C are average of duplicate wells normalized to untreated transfected wells. Exposure of κB-Luc-transfected RAW 264.7 cells to a similar concentration of the control oligonucleotide lacking CpG motifs did not induce an increase luciferase expression (Figures 1B and 1C). κB-dependent luciferase expression in RAW 264.7 macrophages showed similar kinetics in cells treated with a CpG oligonucleotide or with LPS (Figure 1C); both treatments induced a rapid response (greater than two-fold increase by 2 hours) that reached a plateau by 4 to 6 hours.

These results demonstrate that activation of NF-κB pathway by CpG oligonucleotides in RAW 264.7 cells is specific, rapid and dose-dependent. An indirect effect of CpG oligonucleotides mediated by cytokines is unlikely in light of the very rapid kinetics of signaling. Addition of supernatants from CpG oligonucleotide-treated RAW 264.7 cells to κB-Luc-transfected RAW 264.7 macrophages induced little luciferase expression (less than two-fold at 4 hours), confirming that CpG motifs have a direct effect on κB-Luc transcription.

### Example 3: Expression Of A Dominant-Negative MyD88 Blocks CpG-Induction Of κB-Dependent Reporter Gene

In light of the role of Toll receptors in responses to a variety of bacterially derived structures, including LPS, peptidoglycan and lipoproteins, a Toll-related receptor is believed to be involved in signaling in response to oligonucleotides containing unmethylated CpG sequences, which are also characteristic of pathogenic non-self. To this end, RAW 264.7 cells were cotransfected with the kB-luciferase plasmid and an expression vector encoding a dominant-negative form of MyD88, an adapter protein required for signaling by members of the Toll/IL-1 receptor family. The dominant-negative MyD88 used in these experiments, MyD88*lpr,* has an intact Toll homology domain, but contains a point mutation in the death domain (DD). A similar mutation in the death domain of Fas in the *lpr* mouse abrogates Fas signaling, presumably by altering conformation of the death domain, thus blocking association with downstream signaling molecules. Over-expression of MyD88*lpr* in RAW 264.7 cells is expected to interrupt MyD88-dependent signaling by competing with endogenous MyD88 for association with proteins containing Toll homology domains. This effect of *MyD88lpr* is specific to Toll-related proteins, as the mutation in the death should prevent association with downstream signaling components that might be shared with other activators of NF-κB.

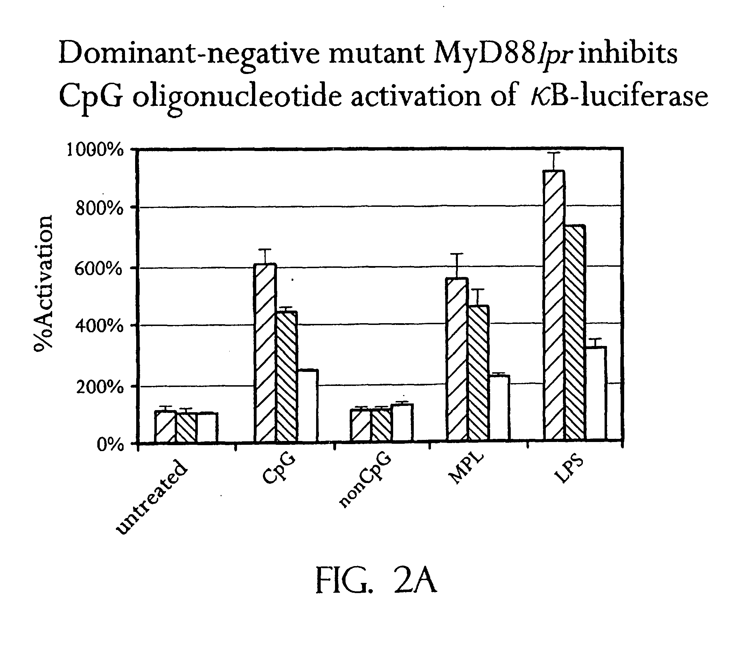

Transfection of RAW 264.7 cells with the plasmid encoding FLAG-tagged MyD88*lpr* resulted in expression of a protein of the expected size, as determined by immunoblotting with an anti-FLAG antibody (data not shown). Regarding Figure 2A, RAW 264.7 cells were then transfected with κB-Luc (1 µg) alone (black bars) or with a plasmid encoding MyD88lpr at two different ratios, 10:1 (gray bars) or 1:1 (white bars). Total plasmid DNA concentration was normalized across all wells by addition of empty vector. Twenty-four hours post-transfection cells were treated with CpG oligonucleotide (4 µM), a control oligonucleotide lacking CpG motifs (4 µM), MPL (1 µg/ml) or LPS (1 µg/ml). Results shown are average +/- standard deviation of duplicate wells normalized to average values of unstimulated transfected controls (untreated columns) and are representative of results obtained in multiple experiments. In RAW 264.7 cells expressing the dominant-negative MyD88*lpr* inhibited κB-dependent luciferase activity induced by CpG oligonucleotide treatment, MPL, or LPS the positive control (Figure 2A). The degree of inhibition luciferase activity by dominant-negative MyD88 depended on the amount of MyD88*lpr* plasmid used in the transfection (Figure 2A) and the resulting level of MyD88*lpr* expression (data not shown). An indirect effect of CpG oligonucleotides mediated by induced expression of IL-18 or IL-1, whose receptors require MyD88 for signal transduction, may account for these results. However, in addition to the negative results obtained when RAW 264.7 macrophages were treated with recombinant IL-18 (see above), no active IL-18 was detectable in RAW264.7 cell extracts or supernatants, by immunoblotting or ELISA, respectively (data not shown).

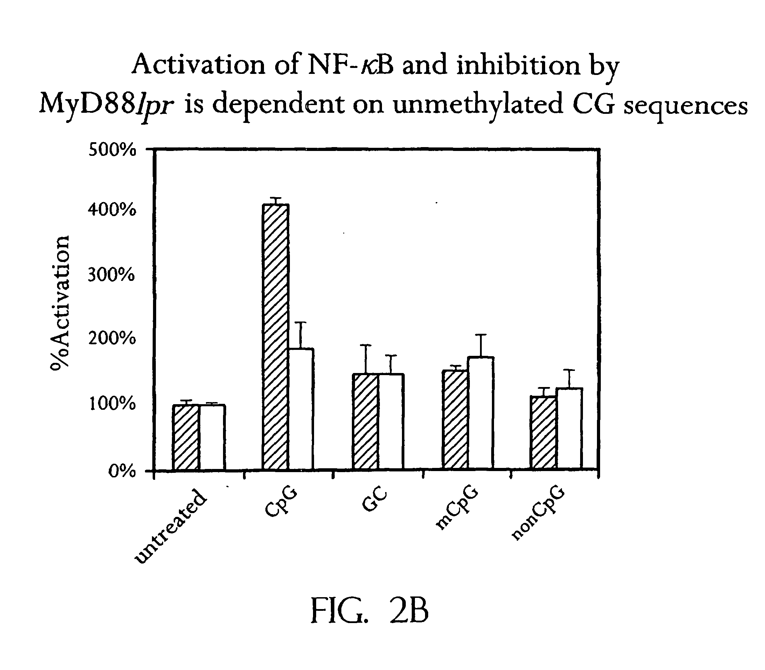

The dependence of the activation of κB-luciferase and inhibition of MyD88lpr on the methylation state and CG dinucleotide sequence in the CpG oligonucleotide was examined. Control oligonucleotides with GC replacing CG sequences (GC oligo) or synthesized with methyl-C rather than. C (methylCG) were added to RAW 264.7 cells transfected with κB-Luc alone or with MyD88*lpr*. In regard to Figure 2B, RAW 264.7 macrophages were transfected with κB-Luc alone (black bars) or κB-Luc plus MyD88*lpr* (white bars) (1:1 plasmid ratio); total DNA concentration was kept constant by addition of empty vector plasmid. Transfected cells were treated the following day for 6 hours with oligonucleotides (4 µM) containing unmethylated CG motifs, similar oligonucleotide with unmethylated GC (GC) or with methylated CG motifs (mCG) replacing the CpG motifs, or an unrelated sequence (non-CpG) lacking CG motifs. Results shown are average +/standard deviation of duplicate wells normalized to average values of unstimulated transfected controls ("untreated" columns) and are representative of results obtained in two experiments. As shown in Figure 2B, methylation of CG sequences or their replacement with GC abrogated nearly all activation of κB-Luc by the oligonucleotides. Basal luciferase activity in cells treated with oligonucleotides lacking CpG motifs was not inhibited by MyD88lpr expression. These results suggest the MyD88-dependent receptor activated by the CpG oligonucleotide has the expected specificity for the unmethylated CG sequences intended to mimic bacterial DNA.

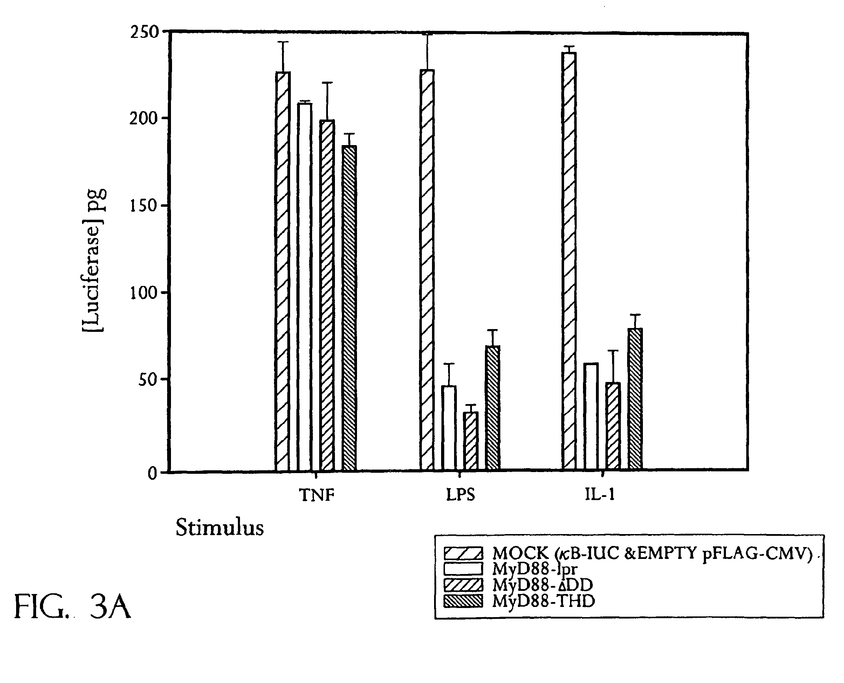

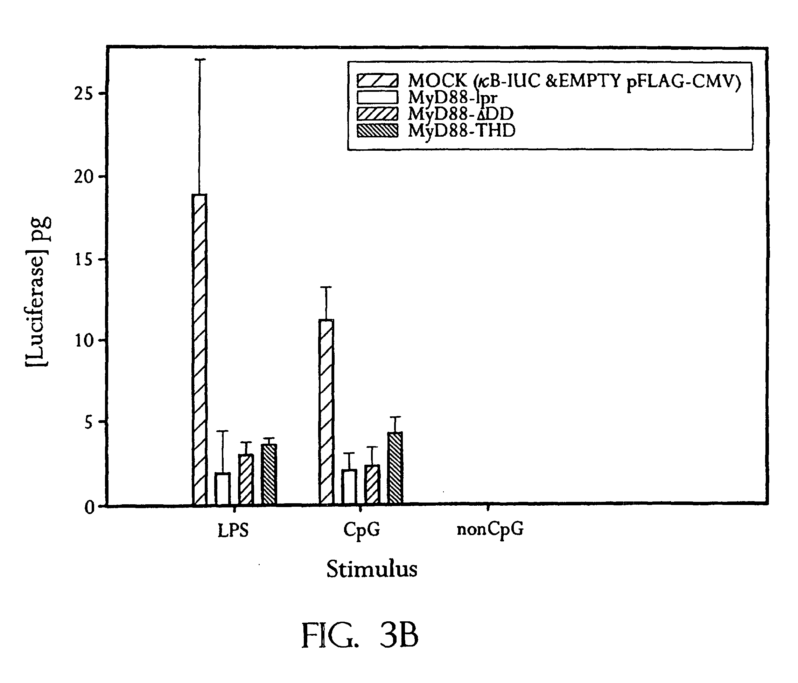

In another set of experiments, three MyD88 constructs lacking the ability to transduce signals were amplified by PCR, and placed under constitutive control of the CMV promoter (pCMV-FLAG vector, Sigma Aldrich). The first construct, MyD88*lpr*, contains a point mutation F56N in the death domain. The second construct, MyD88-ΔDD, lacks the death domain completely. The third construct, MyD88-THD, lacks both the death domain and the intermediate domain. The DNA was transfected into RAW 264.7 cells or murine embryonic NIH-3T3 cells using a cationic lipid reagent. A luciferase reporter plasmid was cotransfected. Possessing an upstream κB0dependent promoter, luciferase transcription was reliant on the release of NF-κB, which is one of the final events in the Toll signaling pathway. Cells were then stimulated with CpG oligonucleotides and several control cytokines. Luciferase expression was assayed on a luminometer to determine if the mutant MyD88 protein had affected CpG signaling. Restriction analysis and sequencing results confirmed the presence of the MyD88 constructs (data not shown). Dominant negative MyD88 specifically and selectively blocks signaling in response to CpG oligonucleotide stimulation (Figures 3A and 3B). The constructs did not affect signaling via the TNF MyD88-independent pathway (negative control), but did inhibit MyD88-dependent LPS signaling and IL-1 signaling (Figure 3A). The MyD88 TIR domain alone was sufficient to block signaling.

These results demonstrate MyD88 function is required for full activation of NF-κB by CpG oligonucleotides and MPL. Since all known receptors that require MyD88 share a common structural feature, THD, this finding also implied that a component of the putative CpG receptor has a THD. The presence of a receptor with a THD and involvement of Toll pathway components in signaling induced by CpG motifs is novel.

This result provides a first insight into the likely structural characteristics of a CpG receptor component. It will be recognized by those skilled in the art that knowing that the immune response to oligonucleotides containing unmethylated CpG motifs is mediated through a receptor containing a THD provides new approaches to identifying the corresponding gene(s). The methylation dependence and fine sequence specificity of the MyD88-dependent effects of CpG oligonucleotides, as well as investigating the relevance of these findings to other nucleotide-based activators of APCs can be performed.

### Example 4: TLR4 Is Not Required For CpG Signal Transduction

A preferred method of determining the polypeptide sequence of the CpG-R is to test whether known TLRs might be likely candidates. A requirement for TLR4 for responses to LPS in mouse has been demonstrated using the endotoxin unresponsive strains C3H/HeJ which has a point mutation in TLR4, and C57B1/10ScCr and C57B1/10ScNCr mice, which do not express TLR4 (Vogel et al., J. Immunol., 1999, 162, 5666-70, Qureshi et al., J. Exp. Med., 1999, 189, 615-25, Chow et al., J. Biol. Chem.,1999, 274, 10689-92, Hoshino et al., J. Immunol.,1999,162, 3749-52, and Poltorak et al., Science, 1998, 282, 2085-8).

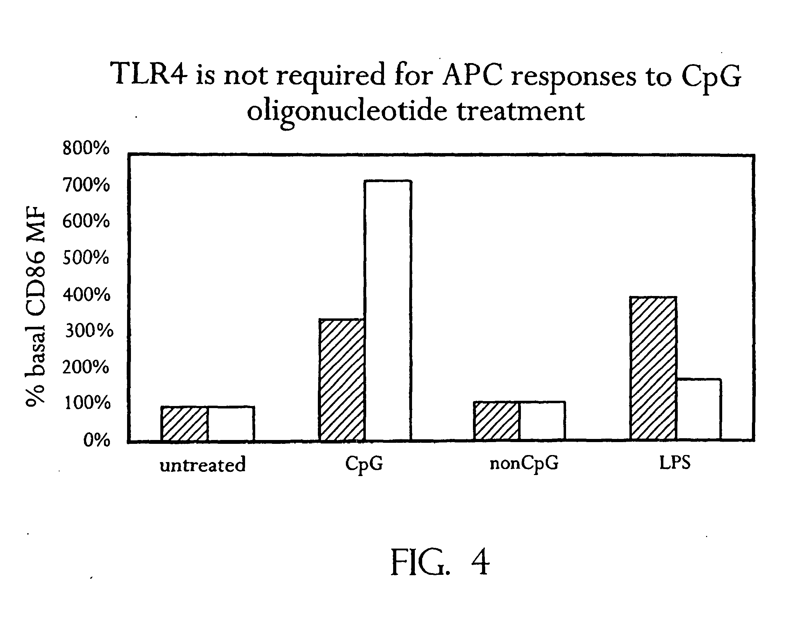

To assess the role of TLR4 in responses of APCs to CpG oligonucleotides and MPL *in vitro,* BMDDC from LPS-responsive mice (Balb/c) and C3H/HeJ mice were cultured overnight with these adjuvants and upregulation of markers of DC activation/maturation was assayed. Cell-surface CD86 expression was quantified by flow cytometry. CD86 is a NF-κB target gene upregulated in activated dendritic cells and macrophages, which enhances their capacity to activate antigen-specific T cells. In regard to Figure 4, BMDDC from wild-type (Balb/c; black bars) and TLR4 mutant mice (C3H/HeJ; white bars) were grown in GM-CSF, as described above, for 6 days and then treated overnight with CpG oligonucleotide (5 µM), a control oligonucleotide (5 µM), or LPS (1 µg/ml), or left unstimulated. Cell surface expression of CD86 on live CD 11c positive cells was assayed by flow cytometry. Results shown are geometric mean fluorescence (MF) of adjuvant-treated BMDDC normalized to MF of untreated BMDDC from the same culture and are representative of results obtained in three experiments. As shown in Figure 4, wild-type BMDDC treated with CpG oligonucleotide, LPS showed increased cell-surface CD86 expression. In TLR4 mutant BMDDC, no change in CD86 expression was observed in MPL- or LPS-treated cells, whereas an increase in CD86 expression similar to that observed in the wild-type BMDDC was seen in CpG oligonucleotide-treated cultures (Figure 4 and data not shown). Thus, TLR4 is required for APC activation *in vitro* by LPS and MPL, but not CpG oligonucleotides.

Further experiments similar to the one above can be carried out to test other known Toll-like receptors to better characterize the precise nature of the CpG-R. This can be carried out in any comparable cell lines having known intact TLRs and mutations that render those receptors non-functional. Identification of the cells with a receptor that is activated by CpG when intact, but not activated when the receptor is defective, will pinpoint precisely which TLR and which particular THD is present.

The results disclosed herein also will be recognized by those skilled in the art to provide useful approaches for developing compounds that may serve as agonists or antagonists of cellular signaling that is mediated by receptors which require the adapter protein MyD88 for their signaling pathways. Compounds can be incubated with cells and assayed to see if the cascade of events known to follow from CpG activation has been effected. The cells can then be transfected with the MyD88*lpr* gene. Compounds that produce the effect in controls without the MyD88*lpr* gene, but fail to do so when the M*y*D88*lpr* gene is expressed will have been shown to activate intracellular signaling via pathways which require the MyD88 adapter protein.

### Example 5: Isolation Of The CpG-R

A number of different procedures can be utilized to identify the Toll-related, CpG-R component. First, one skilled in the art can examine the ability of known and novel Toll-like receptors to confer responsiveness to CpG oligonucleotides on unresponsive cells. Second, one skilled in the art can use MyD88 to purify interacting proteins specifically activated by CpG oligonucleotides and not other Toll receptor ligands, (e.g. LPS), or to isolate the corresponding cDNA using the yeast two-hybrid system.

Mouse Toll-like receptors 1-6 (TLR1-6) have closely related human homologs. Complete or partial sequences are available in public databases for the mouse genes. Expression vectors encoding the TLRs can be transfected into a CpG unresponsive cell line (e.g. NIH3T3) along with a reporter gene activated by CpG oligonucleotides (e.g. κB-luciferase). If any of the cotransfected TLRs confer responsiveness to CpG oligonucleotides, that will be reflected by expression of the reporter gene upon treatment with CpG oligonucleotides.

A similar approach can be used to test the involvement of any candidate CpG-R component in CpG signaling, including novel TLRs. Approaches to identifying and cloning novel Toll-related receptors include genomics, screening sequence databases, degenerate PCR, the yeast two-hybrid system, and library screening by hybridization. Because active CpG oligonucleotides are best described in rodents, not primates, it is preferred to work in the mouse system to identify the Toll-related CpG component, and then isolate a human homolog using, for example, PCR.

MyD88*lpr*, which contains an intact Toll homology domain and a single inactivating point mutation in the death domain can be expressed as a recombinant protein, either in bacteria or in eukaryotic cells. The recombinant protein can be used as an affinity reagent to isolate and purify interacting proteins from CpG oligonucleotide-treated cells (e.g. RAW264.7). Binding of downstream effector molecules in the TLR signaling pathway (e.g. IRAK, TRAF6) should be minimized or eliminated by the *"lpr"* mutation in the death domain. Thus, the protein(s) binding specifically to MyD88*lpr* will be those that interact with the THD and/or the region linking the THD to the death domain. As Toll homology domains associate via homotypic interaction, the CpG-R component containing a THD is expected to bind the MyD88 THD. Once the putative CpG-R has been affinity purified on MyD88, the corresponding cDNA can be isolated by standard methods such as, for example, peptide analysis and PCR.

Similarly, MyD88*lpr* can be used as bait in the yeast two-hybrid system to isolate cDNAs encoding interacting proteins. The relevance of these cDNAs to CpG oligonucleotide-induced signaling can then be tested as described above.

## Claims

1. Use of a polypeptide as a CpG receptor, wherein the polypeptide comprises both a Toll Homology Domain and binds to CpG oligonucleotides.

2. The use of claim 1 wherein the polypeptide can interact with MyD88 protein.

3. The use of claim 1 or claim 2, wherein the polypeptide is a Toll-like receptor (TLR).

4. An antibody which binds to an epitope on a polypeptide, wherein said polypeptide comprises both a Toll Homology Domain and binds to CpG oligonucleotides and wherein the antibody blocks the binding of CpG to said polypeptide, for use as a medicament.

5. The antibody of claim 4, wherein the polypeptide can interact with MyD88 protein.

6. The antibody of claim 4 or claim 5, wherein the polypeptide is a Toll-like receptor (TLR).

7. Use of an antibody which binds to an epitope on a polypeptide, wherein said polypeptide comprises both a Toll Homology Domain and which binds to CpG oligonucleotides, to block the binding of CpG to said polypeptide.

8. Use of claim 7, wherein the polypeptide can interact with MyD88 protein.

9. Use of claim 7 or claim 8, wherein the polypeptide is a Toll-like receptor (TLR).

10. A method of identifying a compound which binds to or modulates an activity of CpG receptor which comprises both a Toll Homology Domain and which binds to CpG oligonucleotides, comprising: contacting said receptor, or cells expressing said receptor, with a compound; and determining whether said compound binds to or modulates the ability of said receptor to bind to CpG oligonucleotides.

11. The method of claim 10, wherein the polypeptide can interact with MyD88 protein.

12. The method of claim 10 or claim 11, wherein the polypeptide is a Toll-like receptor (TLR).

13. A receptor/ligand complex, wherein the receptor is a polypeptide which comprises both a Toll Homology Domain and binds to CpG oligonucleotides and the ligand is CpG oligonucleotide.

14. The complex of claim 13, wherein the polypeptide can interact with MyD88 protein.

15. The complex of claim 13 or claim 14, wherein the polypeptide is a Toll-like receptor (TLR).

## Patentansprüche

1. Verwendung eines Polypeptids als CpG-Rezeptor, wobei das Polypeptid sowohl eine Toll-Homologie-Domäne umfasst als auch an CpG-Oligonukleotide bindet.

2. Verwendung nach Anspruch 1, wobei das Polypeptid mit MyD88-Protein interagieren kann.

3. Verwendung nach Anspruch 1 oder Anspruch 2, wobei das Polypeptid ein Toll-artiger Rezeptor (Toll-like receptor, TLR) ist.

4. Antikörper, der an ein Epitop auf einem Polypeptid bindet, wobei das Polypeptid sowohl eine Toll-Homologie-Domäne umfasst als auch an CpG-Oligonukleotide bindet, und wobei der Antikörper die Bindung von CpG an das Polypeptid blockiert, zur Verwendung als Medikament.

5. Antikörper nach Anspruch 4, wobei das Polypeptid mit MyD88-Protein interagieren kann.

6. Antikörper nach Anspruch 4 oder Anspruch 5, wobei das Polypeptid ein Toll-artiger Rezeptor (Toll-like receptor, TLR) ist.

7. Verwendung eines Antikörpers, der an ein Epitop auf einem Polypeptid bindet, wobei das Polypeptid sowohl eine Toll-Homologie-Domäne umfasst als auch an CpG-Oligonukleotide bindet, zur Blockierung der Bindung von CpG an das Polypeptid.

8. Verwendung nach Anspruch 7, wobei das Polypeptid mit MyD88-Protein interagieren kann.

9. Verwendung nach Anspruch 7 oder 8, wobei das Polypeptid ein Toll-artiger Rezeptor (Toll-like receptor, TLR) ist.

10. Verfahren zur Identifizierung einer Verbindung, die an einen CpG-Rezeptor bindet oder eine Aktivität desselben moduliert, wobei der Rezeptor sowohl eine Toll-Homologie-Domäne umfasst als auch an CpG-Oligonukleotide bindet, bei dem man: den Rezeptor oder Zellen, die den Rezeptor exprimieren, mit einer Verbindung in Kontakt bringt; und bestimmt, ob die Verbindung an den Rezeptor bindet oder die Fähigkeit des Rezeptors zur Bindung an CpG-Oligonukleotide moduliert.

11. Verfahren nach Anspruch 10, wobei das Polypeptid mit MyD88-Protein interagieren kann.

12. Verfahren nach Anspruch 10 oder Anspruch 11, wobei das Polypeptid ein Toll-artiger Rezeptor (Toll-like receptor, TLR) ist.

13. Rezeptor/Ligand-Komplex, wobei der Rezeptor ein Polypeptid ist, das sowohl eine Toll-Homologie-Domäne umfasst als auch an CpG-Oligonukleotide bindet, und der Ligand ein CpG-Oligonukleotide ist.

14. Komplex nach Anspruch 13, wobei das Polypeptid mit MyD88-Protein interagieren kann.

15. Komplex nach Anspruch 13 oder Anspruch 14, wobei das Polypeptid ein Toll-artiger Rezeptor (Toll-like receptor, TLR) ist.

## Revendications

1. Utilisation d'un polypeptide en tant que récepteur de CpG, dans laquelle le polypeptide à la fois comprend un domaine d'homologie Toll et se lie aux oligonucléotides CpG.

2. Utilisation selon la revendication 1, dans laquelle le polypeptide peut interagir avec la protéine MyD88.

3. Utilisation selon la revendication 1 ou la revendication 2, dans laquelle le polypeptide est un récepteur du type Toll (TLR).

4. Anticorps qui se lie à un épitope sur un polypeptide, dans lequel ledit polypeptide à la fois comprend un domaine d'homologie Toll et se lie aux oligonucléotides CpG, et dans lequel l'anticorps bloque la liaison du CpG audit polypeptide, pour une utilisation en tant que médicament.

5. Anticorps selon la revendication 4, dans lequel le polypeptide peut interagir avec une protéine MyD88.

6. Anticorps selon la revendication 4 ou la revendication 5, dans lequel le polypeptide est un récepteur du type Toll (TLR).

7. Utilisation d'un anticorps qui se lie à un épitope sur un polypeptide, dans lequel ledit polypeptide à la fois comprend un domaine d'homologie Toll et se lie aux oligonucléotides CpG, pour bloquer la liaison du CpG audit polypeptide.

8. Utilisation selon la revendication 7, dans laquelle le polypeptide peut interagir avec une protéine MyD88.

9. Utilisation selon la revendication 7 ou la revendication 8, dans laquelle le polypeptideest un récepteur du type Toll (TLR).

10. Procédé d'identification d'un composé qui se lie à ou module une activité de récepteur de CpG, qui à la fois comprend un domaine d'homologie Toll et se lie aux oligonucléotides CpG, comprenant les étapes consistant à : mettre en contact ledit récepteur, ou les cellules exprimant ledit récepteur, avec un composé ; et à déterminer si ledit composé se lie à ou module la capacité dudit récepteur à se lier aux oligonucléotides CpG.

11. Procédé selon la revendication 10, dans lequel le polypeptide peut interagir avec une protéine MyD88.

12. Procédé selon la revendication 10 ou la revendication 11, dans lequel le polypeptide est un récepteur du type Toll (TLR).

13. Complexe récepteur/ligand, dans lequel le récepteur est un polypeptide qui à la fois comprend un domaine d'homologie Toll et se lie aux oligonucléotides CpG, le ligand étant un oligonucléotide CpG.

14. Complexe selon la revendication 13, dans lequel le polypeptide peut interagir avec une protéine MyD88.

15. Complexe selon la revendication 13 ou la revendication 14, dans lequel le polypeptide est un récepteur du type Toll (TLR).


### SEQUENCE LISTING

<110> MacKichan, Mary Lee
<120> CpG Receptor (CpG-R) And Methods Relating Thereto
<130> CHIR-0276
<160> 30
<210> 1
   <211> 20 bases
   <212> DNA
   <213> Artificial Sequence
<220>
   <223> Description of Artificial Sequence: CpG oligonucleotide
<400> 1
   tccatgacgt tcctgacgtt 20
<210> 2
   <211> 20 bases
   <212> DNA
   <213> Artificial Sequence
<220>
   <223> Description of Artificial Sequence: CpG oligonucleotide
<400> 2
   tccaggactt ctctcaggtt 20
<210> 3
   <211> 20 bases
   <212> DNA
   <213> Artificial Sequence
<220>
   <223> Description of Artificial Sequence: CpG oligonucleotide
<400> 3
   tccatgacgt tcctgacgtt 20
<210> 4
   <211> 20 bases
   <212> DNA
   <213> Artificial Sequence
<220>
   <223> Description of Artificial Sequence: CpG oligonucleotide
<400> 4
   ataatcgacg ttcaagcaag 20
<210> 5
   <211> 20 bases
   <212> DNA
   <213> Artificial Sequence
<220>
   <223> Description of Artificial Sequence: CpG oligonucleotide
<400> 5
   ggggtcaacg ttgagggggg 20
<210> 6
   <211> 18 bases
   <212> DNA
   <213> Artificial Sequence
<220>
   <223> Description of Artificial Sequence: CpG oligonucleotide
<400> 6
   tctcccagcg tgcgccat 18
<210> 7
   <211> 20 bases
   <212> DNA
   <213> Artificial Sequence
<220>
   <223> Description of Artificial Sequence: CpG oligonucleotide
<400> 7
   gagaacgctc gaccttcgat 20
<210> 8
   <211> 20 bases
   <212> DNA
   <213> Artificial Sequence
<220>
   <223> Description of Artificial Sequence: CpG oligonucleotide
<400> 8
   tccatgtcgt tcctgatgct 20
<210> 9
   <211> 20 bases
   <212> DNA
   <213> Artificial Sequence
<220>
   <223> Description of Artificial Sequence: CpG oligonucleotide
<400> 9
   tccatgacgt tcctgatgct 20
<210> 10
   <211> 15 bases
   <212> DNA
   <213> Artificial Sequence
<220>
   <223> Description of Artificial Sequence: CpG oligonucleotide
<400> 10
   gctagacgtt agcgt 15
<210> 11
   <211> 20 bases
   <212> DNA
   <213> Artificial Sequence
<220>
   <223> Description of Artificial Sequence: CpG oligonucleotide
<400> 11
   atcgactctc gagcgttctc 20
<210> 12
   <211> 20 bases
   <212> DNA
   <213> Artificial Sequence
<220>
   <223> Description of Artificial Sequence: CpG oligonucleotide
<400> 12
   gaaccttcca tgctgttccg 20
<210> 13
   <211> 15 bases
   <212> DNA
   <213> Artificial Sequence
<220>
   <223> Description of Artificial Sequence: CpG oligonucleotide
<400> 13
   gctagatgtt agcgt 15
<210> 14
   <211> 8 bases
   <212> DNA
   <213> Artificial Sequence
<220>
   <223> Description of Artificial Sequence: CpG oligonucleotide
<400> 14
   tcaacgtt 8
<210> 15
   <211> 8 bases
   <212> DNA
   <213> Artificial Sequence
<220>
   <223> Description of Artificial Sequence: CpG oligonucleotide
<400> 15
   gcaacgtt 8
<210> 16
   <211> 8 bases
   <212> DNA
   <213> Artificial Sequence
<220>
   <223> Description of Artificial Sequence: CpG oligonucleotide
<400> 16
   tcgacgtc 8
<210> 17
   <211> 8 bases
   <212> DNA
   <213> Artificial Sequence
<220>
   <223> Description of Artificial Sequence: CpG oligonucleotide
<400> 17
   tcagcgct 8
<210> 18
   <211> 8 bases
   <212> DNA
   <213> Artificial Sequence
<220>
   <223> Description of Artificial Sequence: CpG oligonucleotide
<400> 18
   tcaacgct 8
<210> 19
   <211> 8 bases
   <212> DNA
   <213> Artificial Sequence
<220>
   <223> Description of Artificial Sequence: CpG oligonucleotide
<400> 19
   tcatcgat 8
<210> 20
   <211> 8 bases
   <212> DNA
   <213> Artificial Sequence
<220>
   <223> Description of Artificial Sequence: CpG oligonucleotides
<400> 20
   tcttcgaa 8
<210> 21
   <211> 22 bases
   <212> DNA
   <213> Artificial Sequence
<220>
   <223> Description of Artificial Sequence: CpG oligonucleotide
<400> 21
   tgactgtgaa cgttcgagat ga 22
<210> 22
   <211> 22 bases
   <212> DNA
   <213> Artificial Sequence
<220>
   <223> Description of Artificial Sequence: CpG oligonucleotide
<400> 22
   tgactgtgaa cgttagcgat ga 22
<210> 23
   <211> 22 bases
   <212> DNA
   <213> Artificial Sequence
<220>
   <223> Description of Artificial Sequence: CpG oligonucleotide
<400> 23
   tgactgtgaa cgttagagcg ga 22
<210> 24
   <211> 22 bases
   <212> DNA
   <213> Artificial Sequence
<220>
   <223> Description of Artificial Sequence: CpG oligonucleotide
<400> 24
   gtttgcgcaa cgttgttgcc at 22
<210> 25
   <211> 22 bases
   <212> DNA
   <213> Artificial Sequence
<220>
   <223> Description of Artificial Sequence: CpG oligonucleotide
<400> 25
   atggcaacaa cgttgcgcaa ac 22
<210> 26
   <211> 22 bases
   <212> DNA
   <213> Artificial Sequence
<220>
   <223> Description of Artificial Sequence: CpG oligonucleotide
<400> 26
   cattggaaaa cgttcttcgg gg 22
<210> 27
   <211> 22 bases
   <212> DNA
   <213> Artificial Sequence
<220>
   <223> Description of Artificial Sequence: CpG oligonucleotide
<400> 27
   ccccgaagaa cgttttccaa tg 22
<210> 28
   <211> 12 bases
   <212> DNA
   <213> Artificial Sequence
<220>
   <223> Description of Artificial Sequence: CpG oligonucleotide
<400> 28
   attgacgtca at 12
<210> 29
   <211> 22 bases
   <212> DNA
   <213> Artificial Sequence
<220>
   <223> Description of Artificial Sequence: CpG oligonucleotide
<400> 29
   ctttccattg acgtcaatgg gt 22
<210> 30
   <211> 19 bases
   <212> DNA
   <213> Artificial Sequence
<220>
   <223> Description of Artificial Sequence: CpG oligonucleotide
<400> 30
   tccatacgtt cctgacgtt 19